# EUROPEAN PATENT APPLICATION

(11) **EP 1 515 148 A2**
(43) Date of publication of application: **16.03.2005**
(21) Application number: 04255504.5
(22) Date of filing: 10.09.2004
(51) Int. Cl.: G01R 31/36

(54) **System and method for providing battery backup power**

(30) Priority: 11.09.2003 US 660285
(71) Applicant: ETHICON ENDO-SURGERY, INC., Cincinnati, OH 45242-2839 (US)
(72) Inventor: Hickle, Randall S., Lubbock, TX 79407 (US)
(74) Representative: Mercer, Christopher Paul

(57) **Abstract**

A battery backup system is provided for use in a sedation and analgesia system. The battery backup system can include a battery, a power source, and a battery controller connected to power source and the battery. The power source can be connected to the battery controller unidirectionally. In addition, the battery can be connected to the battery controller bidirectionally. The battery controller can help determine whether the sedation and analgesia system should be run with the power source or the battery.

## Description

### Field of the Invention

The present invention relates, in general, to battery backup systems and, more particularly, to battery backup systems integrated with medical devices.

### Background of the Invention

It has long been recognized that continuous power delivery to medical devices, in particular, to systems related to anesthesia, is paramount in assuring patient safety. Patients under anesthesia count on the reliability of such systems to function properly in the event of a power outage, an accidental disruption of AC power, or any other episode that leads to disruption of AC power. To this effect, batteries have been used in cooperation with many medical devices so that in the event of AC power loss, those systems will function normally for a sufficient time to ensure patient safety.

Batteries have also been used as a means to provide portability to medical systems where use of those systems is desired in places where AC power may be absent or inadequate, such as in ambulances, homes, and the like. Generally, these devices function in a monitoring capacity, whereby a patient may travel at will, regardless of limitations based on AC power availability. These systems allow patients and/or hospitals to monitor critical patient parameters in a non-intrusive way by eliminating the limitations associated with systems powered solely by AC.

As the speed of medical device development increases, the need has arisen for battery systems that take into account the specific needs of new developments. In particular, sedation and analgesia systems comprising integrated drug delivery and patient monitoring require a battery backup system that accounts for idiosyncrasies related particularly to such a system. For example, a number of systems related to automatic drug infusion have a backup means capable of maintaining drug infusion in the event of an AC power failure. However, these backup means do not account for the integration of drug delivery with such features as, for example, oxygen delivery, associated with integrated sedation and analgesia systems. Existing battery systems are effective in their present capacities, however they fail to take into account the specific needs of sedation and analgesia systems.

Sedation and analgesia systems that integrate patient monitoring systems and drug delivery systems rely on algorithms based on drug delivery and patient physiological response to drug delivery. Such algorithms are used to calibrate drug delivery to meet the specific needs of a patient undergoing sedation and/or analgesia. Procedures generally start by inputting a general drug delivery regimen into the sedation and analgesia system, where the regimen is automatically or manually altered based on the patient's response to the pre-determined regimen. In the event of an AC power loss, data related to patient response and corrections corresponding to the response may be lost. Losing such data often necessitates rebooting the system to re-establish a new drug regimen. The pre-determined drug regimen is often inferior to the altered regimen based on the response of the patient to drug delivery because the patient may be put in danger of over-medication or under-medication while under the pre-determined regimen and before enough patient data is again collected for calibration of the system and altering of the regimen. Though establishing a pre-determined drug regimen is effective at the beginning of a procedure, where a patient is in little danger, the risks of patient consciousness and/or patient over-sedation are far more critical after the procedure has progressed a while. Therefore, the need has arisen for a system and method for providing reliable maintenance of recorded patient response to variable parameters associated with a sedation and analgesia system.

Rebooting a sedation and analgesia system due to an AC power loss may also result in a lag time, where monitoring and/or drug delivery are unavailable, due to necessary start-up times associated with software, hardware, and/or the testing of a fail-safe module. In order to ensure software functionality, sedation and analgesia systems generally provide testing program modules associated with the sedation and analgesia system that are performed before the drug delivery and/or patient monitoring systems are enabled. Though necessary to ensure patient safety, procedures associated with system start-up may endanger a patient if performed during critical times of a medical procedure. Time lapses in monitoring and/or drug delivery may result in patient under-medication and/or the physician missing a critical patient episode that otherwise would have been registered had the system been functioning fully the whole time. It would therefore be advantageous to provide a system and method for maintaining and/or monitoring drug delivery functionality in the event of a primary AC power loss.

### Brief Summary of the Invention

The present invention provides a system and method for providing reliable maintenance of recorded patient response to variable parameters associated with a sedation and analgesia system. The invention further provides a system and method for maintaining and/or monitoring the drug delivery functionality associated with a sedation and analgesia system in the event of a primary AC power loss. More particularly, the invention provides a battery backup system integral with a sedation and analgesia system. The battery backup system according to the present invention is integral with a sedation and analgesia systems that accounts for particular idiosyncrasies associated with sedation and analgesia systems.

The present invention also provides a sedation and analgesia system having an ambulatory capability irrespective of an AC power source. The invention further provides a system and method for providing reliable maintenance of recorded patient response to variable parameters associated with a sedation and analgesia system.

### Brief Description of the Figures

FIGURE 1 illustrates one embodiment of a sedation and analgesia system having a battery backup system in accordance with the present invention.
FIGURE 2 illustrates a more detailed view of one embodiment of a battery backup system in accordance with the present invention.
FIGURE 3 illustrates one embodiment of a method for using a battery backup system integral with a sedation and analgesia system in accordance with the present invention.

### Detailed Description of the Invention

Before explaining the present invention in detail, it should be noted that the invention is not limited in its application or use to the details of construction and arrangement of parts illustrated in the accompanying drawings and description. The illustrative embodiments of the invention may be implemented or incorporated in other embodiments, variations and modifications, and may be practised or carried out in various ways. Furthermore, unless otherwise indicated, the terms and expressions employed herein have been chosen for the purpose of describing the illustrative embodiments of the present invention for the convenience of the reader and are not for the purpose of limiting the invention.

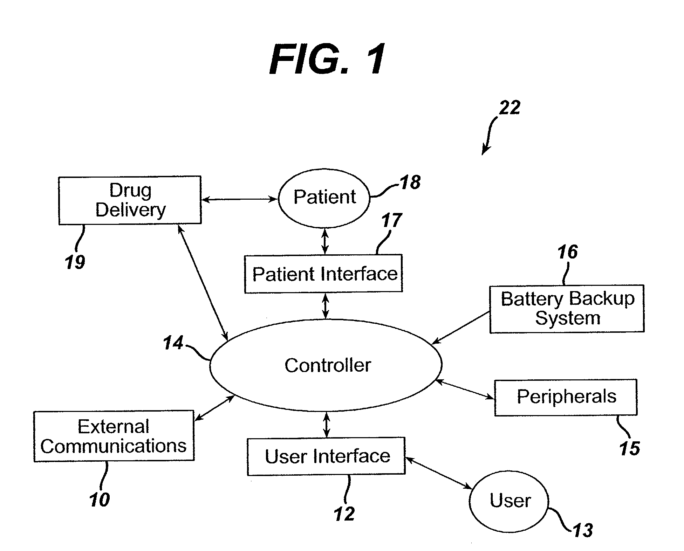

FIGURE 1 illustrates a block diagram depicting one embodiment of the present invention comprising a sedation and analgesia system 22 having user interface 12, such as that described in United States Patent Application Serial No. 60/330,853 filed November 1, 2001 by Hickle, et al, software controlled controller 14, peripherals 15, battery backup system 16, external communications 10, patient interface 17, and drug delivery 19, where sedation and analgesia system 22 is operated by user 13 in order to provide sedation and/or analgesia to patient 18. An example of sedation and analgesia system 22 is disclosed and enabled by United States Patent Application Serial No. 09/324,759, filed June 3, 1999 which is herein incorporated by reference in its entirety.

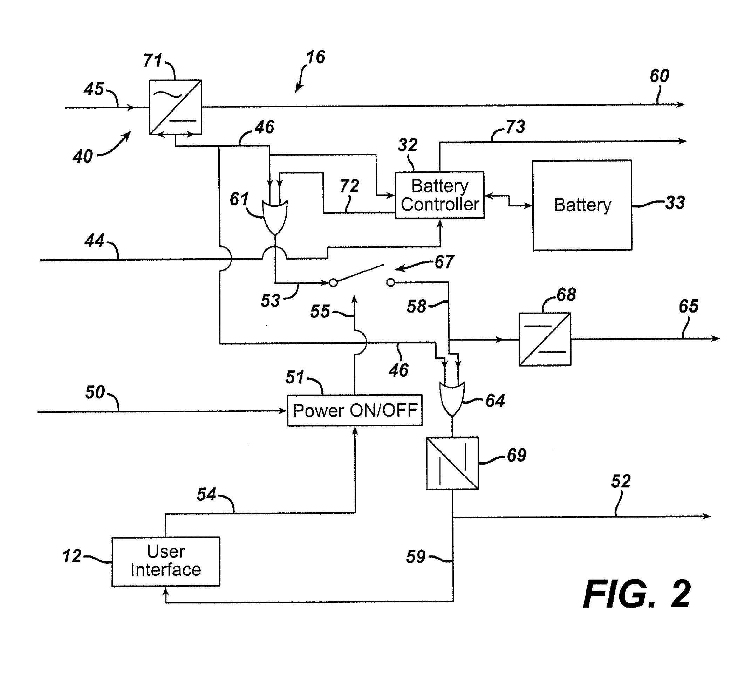

FIGURE 2 illustrates one embodiment of battery backup system 16, where battery backup system 16 comprises of power source 40, which further includes AC power input 45 and AC/DC converter 71, where AC input 45 power is delivered to AC/DC converter 71. AC power input 45 may be, for example, a standard 120 volt wall outlet, however other power sources which produces AC voltages are consistent with the present invention. AC/DC converter 71 may convert for, for example, 120 volts AC to a 28 volt DC output, however other DC voltage outputs are consistent with the present invention. DC output from AC/DC converter 71 is herein referred to as first DC power supply 46. Battery backup system 16 further comprises AC present output 60, where AC present output 60 is a signal transmitted to controller 14 indicating that AC power input 45 is present and/or that AC power input 45 carries sufficient voltage to maintain full functionality of sedation and analgesia system 22.

Power source 40 further includes DC power supply 46. DC power supply 46 is, in one embodiment of the present invention, supplied to battery controller 32, where battery controller 32 comprises a DC/DC converter (not shown) as well as a battery charger (not shown). DC power supply 46 may be passed through the DC/DC converter, where the voltage is stepped down from, for example, the 28 volts associated with DC power supply 46 to 26.3 volts, where the stepped down voltage is used to charge battery 33 via the battery charger. The battery charger may be a current limiting power supply, where current is held constant by altering the voltage of the output of the DC/DC converter associated with battery controller 32. Current delivered to battery 33 from the battery charger may be held constant until battery 33 is fully charged, where the voltage output associated with the DC/DC converter may then be held constant. Battery 33 may be a lithium ion battery, sealed lead battery, or other suitable means of providing a backup DC power source for sedation and analgesia system 22. The present invention comprises modifying the DC/DC converter and/or the battery charger associated with battery controller 32 to account for the particular idiosyncrasies of a variety of batteries, where any suitable battery may be used with sedation and analgesia system 22. Battery 33 further features any suitable charge life, where battery 33 may be configured differently for use with a portable or in-house sedation and analgesia system 22.

DC power may be output from battery 33 via second DC power supply 72, where second DC power supply may be routed through battery controller 32 to first OR logic gate 61. First OR logic gate 61 may be an OR logic gate, and oring diode pair, or other suitable electrical juncture. Battery controller 32, in one embodiment of the present invention, comprises a current sensor, where the DC voltage used to charge battery 33 and the DC voltage output of battery 33 may be monitored. Data related to remaining battery 33 charge, battery 33 charging, battery 33 output, and/or estimated battery life may be output via battery controller output 73, where battery controller output 73 transmits data to controller 14. The present invention further comprises battery communications signal 44, where battery communications signal 44 comprises inputting data to battery controller 32 related to battery profiles, calibration constants, or inputting other data necessary to properly charge and/or operate battery 33.

First OR logic gate 61, in one embodiment of the present invention, allows for either first DC power supply 46 or second DC power supply 72 to deliver primary power to sedation and analgesia system 22. If sufficient AC power is available from AC power input 45, the voltage of first DC power supply 46 will be greater than that of second DC power supply 72, and first DC power supply 46 will act as the primary power source for sedation and analgesia system 22. If sufficient AC power is not available from AC power input 45, second DC power supply 72 may act as the primary power supply for sedation and analgesia system 22.

Battery backup system 16 further comprises power supply 53, where power supply 53 may be either power from first DC power supply 46 or second DC power supply 72. Power supply 53 interfaces with on/off switch 67, where on/off switch 67 allows the delivery of power from power supply 53 to power sedation and analgesia system 22 in the event that sedation and analgesia system 22 is operating properly, and to disallow the delivery of power from variable power supply 53 to sedation and analgesia system 22 in the event that sedation and analgesia system 22 is not functioning properly. On/off switch 67 may be a solid state switch, a relay, a solid state relay, a mosfet, or any other suitable means of controlling power delivery from variable power supply 53 to sedation and analgesia system 22.

On/off switch 67 may be turned off in the event that a software failure, hardware failure, or other potentially dangerous episode occurs or by the discretion of user 13. Such a failure may be indicated to power on/off device 51 via signals 50 from a fail safe module, a software health check monitor, or from any other source monitoring the functionality of sedation and analgesia system 22 or via signal 54 from user interface 12 programmed by user 13. Signals 50 may be binary transmissions, analog transmissions, or both. Power on/off device 51 may be a programmable controller, a microprocessor, a series of logic gates, or any other suitable means of receiving signals from sedation and analgesia system 22 and turning on/off switch 67 off in the event of a sedation and analgesia system malfunction. Power on/off device 51 may turn on/off switch 67 on or off via actuator signal 55, where actuator signal 55 may be a transistor-transistor logic (TTL) signal and on/off switch 67 is a mosfet. At the beginning of a medical procedure, power supply 53 may pass through on/off switch 67, where a TTL actuator signal 55 is normally present at startup in a properly functioning sedation and analgesia system. The interface of the TTL actuator signal 55 interfaced with on/off switch 67, in the form of a mosfet, allows power from variable power supply 53 to pass through on/off switch 67 as long as the high TTL signal is present. In the event that signals 50 indicate a sedation and analgesia malfunction, power on/off device 51 may drop the voltage of actuator signal 55, thereby disabling power delivery across the mosfet. Other embodiments and combinations of on/off switch 67 and actuator signal 55 are contemplated and are consistent with present invention.

In one embodiment of the present invention, power supply 58 comprises current that has passed on/off switch 67, where power supply 58 may flow to DC/DC converter 68 and/or to second OR logic gate 64. DC/DC converter 68 may, for example, convert the 28 volt power associated with power supply 58 to 12 volts, 5 volts, or any other suitable voltage necessary to run hardware and/or software associated with sedation and analgesia system 22. The present invention comprises a plurality of DC/DC converters, where the voltage of power supply 58 may be stepped down to a plurality of different voltages by the DC/DC converters, where output 65 is the appropriate voltage for one or a plurality of systems such as, for example, patient interface 17, associated with sedation and analgesia system 22.

Second OR logic gate 64 is, in one embodiment of the present invention, an oring diode pair, where second OR logic gate 64 receives power supply 58 and power supply 46 as inputs. The power supply input having the highest voltage will, in one embodiment of the present invention, pass through second OR logic gate 64 to DC/DC converter 69, where power supply 46 or power supply 58 originating from AC power input 45 will generally be dominant with respect to power supply 58 originating from battery 33.

The present invention further comprises DC/DC converter 69, where DC/DC converter 69 may convert the DC power passing through second OR logic gate 64 to a suitable voltage needed to power user interface 12 via interface signal 59. DC/DC converter 69 may also provide the necessary voltages for basic software and/or hardware functionality associated with a sedation and analgesia system 22 in standby mode via basic power signal 52. In one embodiment of the present invention, when on/off switch 67 is turned off by power on/off device 51, sedation and analgesia system 22 may still retain enough power in user interface 12 and/or other basic system functions in order to allow sedation and analgesia system 22 to be rebooted. DC/DC converter 69 may convert power supply 46 or power supply 58 to any suitable voltage such as, for example, 5 volts. The present invention further comprises a plurality of DC/DC converters, where the DC/DC converters may provide any suitable voltage to power any software and/or hardware associated with the standby or power down mode of sedation and analgesia system 22. For example, in the event of a system malfunction, power on/off device 51 may disable the delivery of power from variable power supply 53 to sedation and analgesia system 22, however software associated with sedation and analgesia system 22 may need a brief period of time while under power to properly shut down. Therefore, in one embodiment of the present invention, basic power signal 52 provides sufficient power to insure the safe power down of hardware and/or software associated with sedation and analgesia system 22.

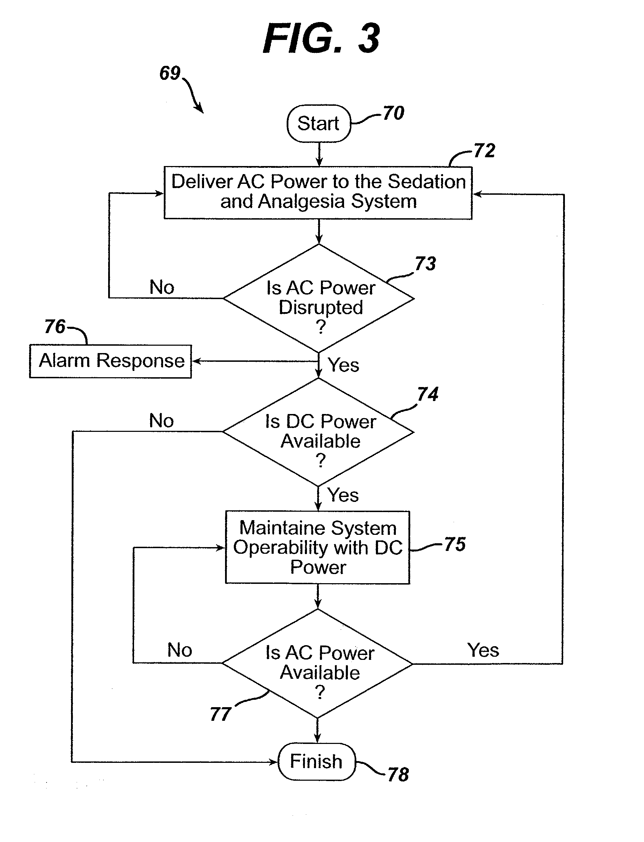

FIGURE 3 illustrates one embodiment of method 69 for employing battery integrated with sedation and analgesia system 22. Method 69 comprises start step 70, herein referred to as step 70, where step 70 comprises providing a battery 33 integral with sedation and analgesia system 22. In one embodiment of the present invention, step 72 of method 69 comprises delivering AC power input 45 to sedation and analgesia system 22, where AC power input 45 may be, for example, a standard 120V wall outlet, however other AC power inputs are consistent with the present invention. The present invention further comprises charging battery 33 via battery controller 32 when AC power input 45 is present.

Method 69 further comprises query 73, where query 73 comprises ascertaining whether AC power input 45 is present throughout the duration of a medical procedure. If AC power input 45 is not disrupted, method 69 will proceed to step 72 and sedation and analgesia system 22 will continue to run on AC power input 45. If AC power input 45 is disrupted, method 69 may proceed to step 76, where step 76 comprises an alarm response to the power disruption. The alarm response of step 76 may be a visual indicator of an AC power input 45 disruption, an audio indicator of an AC power input 45 disruption, and/or any other suitable means of notifying user 13 of the power disruption. Alarm response 76 may be provided to user 13 via user interface 12.

If AC power input 45 is disrupted, method 69 may also proceed to query 74, where query 74 comprises ascertaining whether DC power is available from battery 33. If DC power is not available from battery 33 due to insufficient charge or malfunction, method 69 may proceed to finish step 78. If sufficient DC power is present, method 69 may proceed to step 75, where step 75 comprises maintaining the operability of sedation and analgesia system 22 with DC power from battery 33. If DC power is available, the present invention further comprises alerting user 13 to the estimated charge life remaining in battery 33 and/or other factors relating to the functionality of battery 33. Step 75 further comprises maintaining the functionality of sedation and analgesia system 22 in variable modes, where battery backup system 16 may be designed to provide user 13 with sufficient time to ensure patient 18 safety in the event of a disruption in AC power input 45. For example battery 33 may have a full charge life of ten minutes, where battery 33 maintains full functionality of sedation and analgesia system 22 for five of the ten minutes. Following the initial five minute period, sedation and analgesia system 22 may have only moderate functionality such as, for example, patient monitoring, where drug delivery 19 has been disabled. Providing multiple battery modes allows battery 33 to be small in size while enabling user 13 to ensure patient 18 safety. The present invention comprises providing a plurality of modes of battery 33 operation, where battery 33 may be configured for use in portable sedation and analgesia systems, where the charge life of battery 33 must be substantially longer, or for in-house sedation and analgesia systems, where any suitable battery 33 with any suitable charge life may be provided.

In one embodiment of the present invention, method 69 further comprises query 77, where query 77 comprises ascertaining whether AC power input 45 has become available following at least one disruption of AC power input 45. In one embodiment of the present invention, if AC power input 45 becomes available following a disruption in AC power input, method 69 will proceed to step 72, where sedation and analgesia system 22 will run on AC power input 45. If method 69 proceeds to step 72 from query 77, AC power input 45 may also be used to charge battery 33 in the event a second power outage or other disruption in AC power input 45 occurs. If AC power input 45 is not available, method 69 may proceed to step 75, where sedation and analgesia system 22 may be maintained for any suitable duration by battery 33 or any other suitable DC power source.

In a further embodiment of the present invention, if AC power input 45 is disrupted, method 69 will not proceed to step 72, where user 13 relies on the DC power from battery 33 to insure patient safety before the charge of battery 33 dissipates. Once the charge life of battery 33 has expired, method 69 may proceed to finish step 78, where step 78 comprises the deactivation of all systems associated with sedation and analgesia system 22.

It is further contemplated that certain embodiments of sedation and analgesia system 22 may be used in a portable capacity, where sedation and analgesia system 22 may rely on solely on DC power to maintain system functionality. In such portable embodiments, the remaining charge life of battery 33 may be shown to user 13 throughout the duration of the procedure. Battery 33 may also be configured into a plurality of modes to insure patient 18 safety such as, for example, where battery backup system 16 displays critical warning alarms when the charge life of battery 33 drops below a critical level. Battery 33 of a portable sedation and analgesia system 22 may be recharged by AC power input 45. Battery 33 may also or instead be replaceable.

While the present invention has been illustrated by description of several embodiments, it is not the intention of the applicant to restrict or limit the spirit and scope of the appended claims to such detail. Numerous variations, changes, and substitutions will occur to those skilled in the art without departing from the scope of the invention. Moreover, the structure of each element associated with the present invention can be alternatively described as a means for providing the function performed by the element. Accordingly, it is intended that the invention be limited only by the spirit and scope of the appended claims.

## Claims

1. A battery backup system for a sedation and analgesia system, said battery backup system comprising:
a. a battery;
b. a power source; and
c. a battery controller connected to said power source and said battery wherein said battery controller determines selection of said battery or said power source.

2. The battery backup system of claim 1 wherein said battery is made of lithium ion.

3. The batter backup system of claim 1 or claim 2 wherein said power source further comprises of an AC power input, an AC/DC converter, and a DC power supply.

4. The battery backup system of claim 3 wherein said AC power input is a 120V wall outlet.

5. The battery backup system of claim 3 wherein said AC/DC converter changes said AC power input to said DC power supply.

6. The battery backup system of any one of claims 1 to 5 wherein said power source is connected unidirectionally to said battery controller and said battery is connected bidirectionally to said battery controller.

7. A method of supplying power to a sedation and analgesia system which comprises:
a. supplying power to said sedation and analgesia system from a power source;
b. checking said power source for a disruption;
c. supplying power to said sedation and analgesia system from a battery if said disruption occurs;
d. switching back to said power source from said battery if said disruption is resolved.

8. A method of supplying power to a sedation and analgesia system recited in claim 7 wherein checking said power source for a disruption further includes sounding an alarm if said disruption occurs.

9. A method of supplying power to a sedation and analgesia system recited in claim 7 or claim 8 wherein supplying power to said sedation and analgesia system from a battery if said disruption occurs further includes checking said battery source for availability.

10. A method of supplying power to a sedation and analgesia system recited in claim 9 wherein checking said battery source for availability further includes shutting down said sedation and analgesia system if said battery source is unavailable.
